# EUROPEAN PATENT APPLICATION

(11) **EP 4 645 267 A1**
(43) Date of publication of application: **05.11.2025**
(21) Application number: 23911957.1
(22) Date of filing: 21.12.2023
(51) Int. Cl.: G06V 40/00, A61B 5/1171, A61B 5/1172, G06T 7/00

(54) **BIOLOGICAL INFORMATION ACQUISITION DEVICE**

(30) Priority: 27.12.2022 JP 2022210339
(71) Applicant: Panasonic Intellectual Property Management Co., Ltd., Kadoma-shi, Osaka 571-0057 (JP)
(72) Inventor: NAKAMURA, Hideyuki, Kadoma-shi, Osaka 571-0057 (JP); TANAHASHI, Satoru, Kadoma-shi, Osaka 571-0057 (JP); SHIKANAI, Masaki, Kadoma-shi, Osaka 571-0057 (JP); HORIKI, Toshio, Kadoma-shi, Osaka 571-0057 (JP)
(74) Representative: Eisenführ Speiser
(86) International application number: PCT/JP2023/046035
(87) International publication number: WO 2024/143177

(57) **Abstract**

A biological information acquisition device according to the present invention comprises: a housing; a first imaging unit that captures an image of a first biological site in a non-contact state; a second imaging unit that captures, in a non-contact state, an image of a second biological site different from the first biological site; and an acquisition unit that acquires a first captured image which has been captured by the first imaging unit and a second captured image which has been captured by the second imaging unit, wherein the housing includes a first opening that forms an imaging space for capturing an image of the second biological site, an illumination instrument that illuminates the imaging space inside the housing, and an optical path changing unit that changes the optical path of light reflected by the second biological site in the imaging space to enter the light into the second imaging unit.

## Description

### TECHNICAL FIELD

The present disclosure relates to a biometric information acquisition device.

### BACKGROUND ART

Patent Literature 1 discloses a contactless multiple body part recognition method in which, when a user is sensed within a predetermined measurable range, a plurality of pieces of biometric data are acquired from the user in a contactless state, and then the acquired plurality of pieces of biometric data are compared with previously registered biometric data to perform user authentication on each piece of biometric data. In the contactless multiple body part recognition method, in the user authentication on each piece of biometric data, when the user approaches, the biometric data on a face and an iris is acquired from the user in the contactless state by physical movement of a camera, the user authentication result on the biometric data are integrated, and when the user authentication result on each piece of biometric data satisfies a predetermined condition, the user is determined as an authorized user.

### CITATION LIST

### PATENT LITERATURE

Patent Literature 1: JP2020-510259A

### SUMMARY OF INVENTION

### TECHNICAL PROBLEM

In a configuration of Patent Literature 1, in order to acquire each piece of biometric data necessary for the user authentication, a dedicated camera corresponding to each piece of biometric data is required, and thus manufacturing cost of a multiple body part recognition device is high. Therefore, it is required to realize a contactless biometric authentication system with reduced manufacturing cost by using a highly versatile device.

The present disclosure has been made in view of the above-described circumstances of the related art, and an object of the present disclosure is to provide a biometric information acquisition device that assists in acquisition of a plurality of different pieces of biometric information suitable for biometric authentication in a contactless state.

### SOLUTION TO PROBLEM

The present disclosure provides a biometric information acquisition device including a housing, a first imaging unit configured to capture an image of a first body part of a subject in a contactless state, a second imaging unit configured to capture an image of a second body part different from the first body part of the subject in the contactless state, and an acquisition unit configured to acquire a first captured image captured by the first imaging unit and a second captured image captured by the second imaging unit, in which the housing includes a first opening portion that is formed by cutting out a part of the housing to form an imaging space in which the image of the second body part is captured, a lighting that illuminates the imaging space inside the housing, and an optical path changing unit that changes an optical path of light reflected by the second body part inside the imaging space and causes the light to be incident on the second imaging unit.

### ADVANTAGEOUS EFFECTS OF INVENTION

According to the present disclosure, it is possible to assist in acquisition of a plurality of different pieces of biometric information suitable for biometric authentication in a contactless state.

### BRIEF DESCRIPTION OF DRAWINGS

FIG. 1 is a diagram illustrating an example of a use case of a biometric authentication device according to Embodiment 1;
FIG. 2 is a right side perspective view of the biometric authentication device;
FIG. 3 is a diagram illustrating an example of an opening portion and a housing internal space of the biometric authentication device;
FIG. 4 is a left side perspective view of the biometric authentication device;
FIG. 5 is a block diagram illustrating an internal configuration example of the biometric authentication device according to Embodiment 1;
FIG. 6 is a sequence diagram illustrating an operational procedure example of a terminal device according to Embodiment 1;
FIG. 7 is a diagram illustrating an overall configuration example of a biometric authentication system according to Embodiment 2;
FIG. 8 is a block diagram illustrating an internal configuration example of the biometric authentication system according to Embodiment 2; and
FIG. 9 is a sequence diagram illustrating an operational procedure example of the biometric authentication system according to Embodiment 2.

### DESCRIPTION OF EMBODIMENTS

### (Background of Present Disclosure)

In the related art, there is a method of performing acquisition of biometric information on a plurality of different body parts (for example, a hand, a face, and the like) in a contactless manner using a highly versatile terminal device (for example, a smartphone, a tablet terminal, and the like). In such a case, the terminal device captures an image of the face of a user with a front camera provided on the front side (a monitor side) of the terminal device and captures an image of the hand of the user with a back camera provided on a back side (opposite side to the monitor) of the terminal device, thereby acquiring (capturing) biometric information on each body part in a contactless state.

However, the terminal device may not be able to acquire an image suitable for acquisition of biometric information due to difficulty in focus control on a body part. In particular, the front camera has a wider angle than the back camera, and distortion of the captured image is large. Therefore, it is more difficult for the terminal device to control the focus of the front camera, and it is difficult to acquire an image suitable for acquiring the biometric information.

In addition, when a store clerk of a store or the like acquires biometric information on a plurality of different body parts of a user using the terminal device, the store clerk does not know an appropriate imaging position of the body part (the hand, the face, or the like) of the user with respect to the terminal device, and it is difficult to acquire (capture) the biometric information on the user in the contactless state. In addition, when a store clerk or a user captures an image of a face of the user with a front camera and an image of a finger of the user with a back camera in a store, there is a problem in that light of lighting (light-emitting diode (LED)) provided near the back camera enters eyes of the store clerk and the store clerk feels dazzled.

Therefore, in each embodiment described below, an example of a biometric information acquisition device that assists in acquisition of a plurality of different pieces of biometric information suitable for biometric authentication in a contactless state will be described.

Hereinafter, embodiments specifically disclosing configurations and operations of a biometric information acquisition device according to the present disclosure will be described in detail with reference to the drawings as appropriate. However, unnecessarily detailed description may be omitted. For example, the detailed descriptions of well-known matters and the redundant description of substantially the same configuration may be omitted. This is to avoid unnecessary redundancy of the following description and facilitate understanding of those skilled in the art. The accompanying drawings and the following description are provided for those skilled in the art to fully understand the present disclosure, and are not intended to limit the subject matters described in the claims.

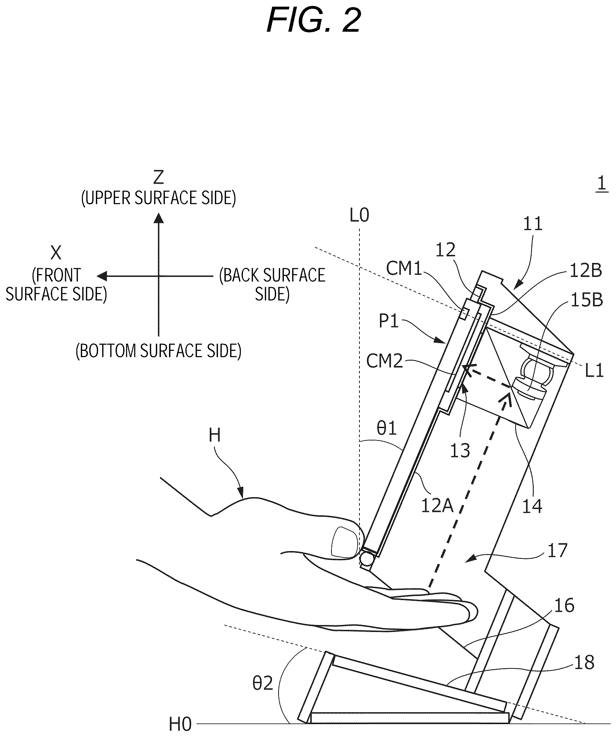

In the following description, an XY plane indicated by an X direction and a Y direction indicates an installation surface H0 (a horizontal plane) of a biometric authentication device 1 (see FIG. 2), and may be referred to as a left-right direction or a front-rear direction. A Z direction indicates a direction perpendicular to the installation surface H0 (the horizontal surface) of the biometric authentication device 1, and may be referred to as an upper-lower direction.

### (Embodiment 1)

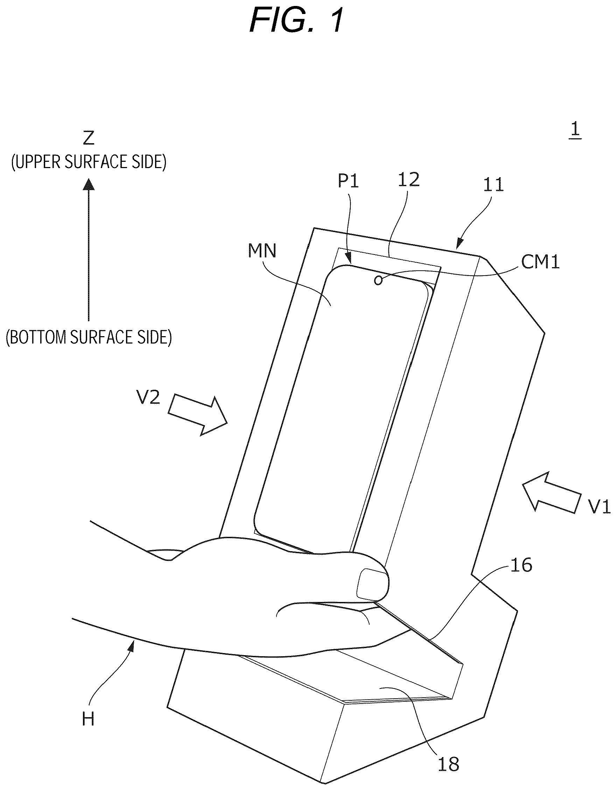

An example of a use case of a biometric authentication device 1 according to Embodiment 1 will be described with reference to FIG. 1. FIG. 1 is a diagram illustrating the example of a use case of the biometric authentication device 1 according to Embodiment 1.

The biometric authentication device 1 (an example of a biometric information acquisition device) according to Embodiment 1 includes a terminal device P1 (an example of an imaging device) and a housing 11. The biometric authentication device 1 is installed in a store that uses biometric authentication, acquires biometric information on a user, registers the acquired biometric information on the user, and performs user authentication based on the acquired biometric information on the user.

The housing 11 has a recess 12 in which the terminal device P1 is detachably placed and an opening portion 16 into which a hand H of the user can be inserted. The housing 11 includes an optical system 14 and at least one lighting 15 inside the housing 11. The housing 11 illuminates the hand H of the user inserted into the opening portion 16, and causes the light reflected by the hand H of the user to be incident on a second camera CM2 of the terminal device P1 by the optical system 14.

The terminal device P1 includes at least two cameras (the first camera CM1 and a second camera CM2 (see FIG. 2)), and is realized by, for example, a smartphone, a tablet terminal, or the like. The terminal device P1 images an image of each of a plurality of different body parts of the user in a state of being placed in the recess 12 of the housing 11.

The terminal device P1 registers a captured image (the biometric information), and performs user authentication using an acquired captured image (the biometric information). The terminal device P1 generates a notification screen (not illustrated) notifying a user authentication result and displays the notification screen on a monitor MN.

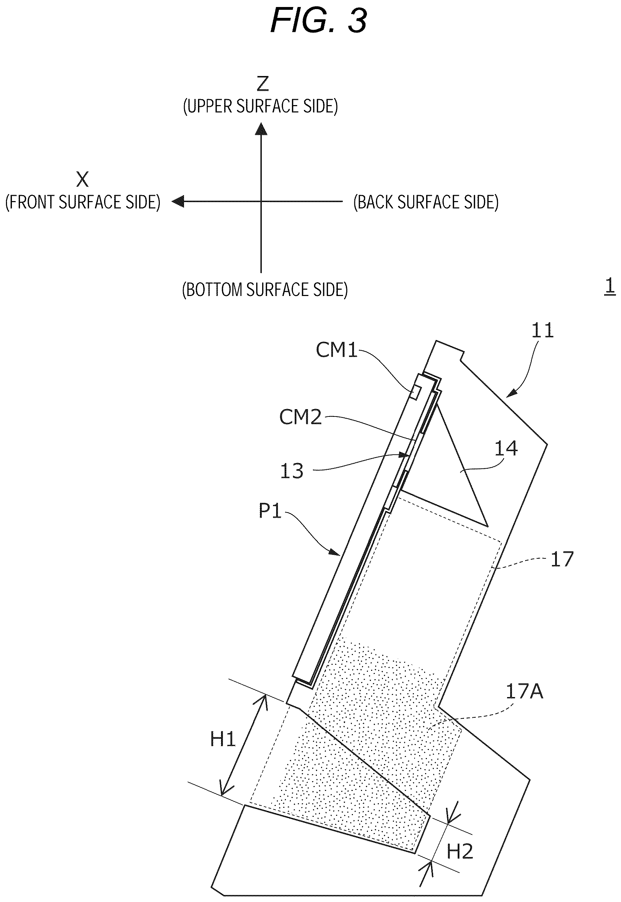

Next, an internal structure of the housing 11 will be described with reference to FIGS. 2 and 3. FIG. 2 is a right side perspective view of the biometric authentication device 1. FIG. 3 is a diagram illustrating an example of the opening portion 16 and a housing internal space 17 of the biometric authentication device 1. The biometric authentication device 1 illustrated in FIGS. 2 and 3 is a perspective view of the inside of the housing 11 of the biometric authentication device 1 viewed from an arrow V1 illustrated in FIG. 1.

The housing 11 includes the recess 12, an opening 13, the optical system 14, lightings 15A and 15B, and the opening portion 16. The configuration of the housing 11 and the arrangement of each configuration are not limited thereto. The housing 11 may be configured to place and hold the terminal device P1 and can image the finger of the user by the second camera CM2 of the terminal device P1.

The housing 11 has the recess 12 (an example of an attachment and detachment portion) formed to be inclined by a first predetermined angle θ1 with respect to a vertical direction L0 (the Z direction). The recess 12 holds the terminal device P1 and has the opening 13 communicating with the inside of the housing 11 on a back surface side of the terminal device P1 where the second camera CM2 is located.

The first predetermined angle θ1 is set to, for example, 23°. The first predetermined angle θ1 is not limited thereto, and may be set to an angle (for example, 15° to 23°) at which the first camera CM1 can capture an image of the face of the user. Accordingly, an optical axis L1 of the first camera CM1 is inclined by the first predetermined angle θ1 with respect to the installation surface H0 of the housing 11.

The opening 13 as an example of a second opening portion is formed by cutting out a part of the recess 12 that is a placement surface of the terminal device P1. The opening 13 allows the second camera CM2 to capture an image of the inside of the housing 11 in a state in which the terminal device P1 is placed in the recess 12. The opening 13 may be formed at a position and with a size corresponding to a position of the second camera CM2 of the terminal device P1.

The housing 11 includes the optical system 14 and the lightings 15A and 15B inside the housing.

The lightings 15A and 15B are inserted from the opening portion 16 and illuminate the finger of the user placed in the housing internal space 17.

The optical system 14 as an example of an optical path changing unit is realized using, for example, a prism, a mirror, or the like. The optical system 14 reflects or refracts light, which is emitted by the lightings 15A and 15B and reflected by the finger of the user, by 90°, and causes the light to be incident on the second camera CM2 through the opening 13.

When the optical system 14 is realized by a prism, the optical system 14 is disposed on a back surface of the installation surface of the terminal device P1 in the recess 12. Since the optical system 14 is disposed such that a distance between the optical system 14 and the second camera CM2 of the terminal device P1 placed in the recess 12 is 0 (zero), it is possible to more effectively prevent reflection of the illumination light of the lightings 15A and 15B.

On the other hand, when the optical system 14 is realized by a mirror, the optical system 14 may be disposed at a position where the optical system 14 can reflect light reflected by the finger of the user at an angle of 90° and cause the light to be incident on the second camera CM2 through the opening 13.

Here, formation positions of the opening 13 and the optical system 14 will be described. In order to acquire a captured image (hereinafter, referred to as a "finger image") in which a finger of the user (for example, a vein, a fingerprint, a palm print, or the like) is in focus, it is desirable that the housing 11 is designed such that a depth of field (an imaging distance) of the second camera CM2 overlaps with an imaging region 17A in the housing internal space 17 into which the finger of the user is inserted as illustrated in FIGS. 2 to 4. That is, the opening 13 is formed such that an optical path length of the incident light reflected by the optical system 14 from the finger of the user and incident on the second camera CM2 is at the imaging distance within the depth of field of the second camera CM2 (for example, within the depth of field) that allows the capture of a finger image suitable for the biometric authentication. Therefore, in Embodiment 1, an example is illustrated in which each of the opening 13 and the optical system 14 is formed on an upper portion (upper surface side) of the recess 12, but the present disclosure is not limited thereto.

The opening portion 16 as an example of a first opening portion is formed from the front surface to both side surfaces of the housing 11 and communicates with the housing internal space 17 inside the housing 11. The opening portion 16 is formed such that an opening height H1 on a front surface side into which the user inserts the hand H is larger than an opening height H2 on the back surface side. Thus, the user can easily insert the hand H from an insertion opening (that is, the front surface side of the housing 11) into which the hand H of the user is inserted toward the housing internal space 17 (that is, the back surface side of the housing).

The housing internal space 17 is a space into which the fingertip of the user as an imaging target can be inserted. The housing internal space 17 includes the imaging region 17A (space) which is a region (space) corresponding to the depth of field of the second camera CM2 of the terminal device P1.

The imaging region 17A as an example of an imaging space is a region (space) determined based on the depth of field of the second camera CM2 and the position of the optical system 14. It is needless to say that the imaging region 17A illustrated in FIG. 3 is an example and the present disclosure is not limited thereto.

An opening lower surface 18 is a lower surface of a space into which the hand H of the user can be inserted through the opening portion 16. The opening lower surface 18 is formed to be inclined by a second predetermined angle θ2 toward the front surface side of the housing 11 in order to guide an insertion direction of the hand H of the user into the housing internal space 17.

As described above, by holding the terminal device P1 in a state of being inclined by the first predetermined angle θ1, the housing 11 can simultaneously or continuously perform imaging of the face of the user and imaging of the fingertip of the hand H of the user.

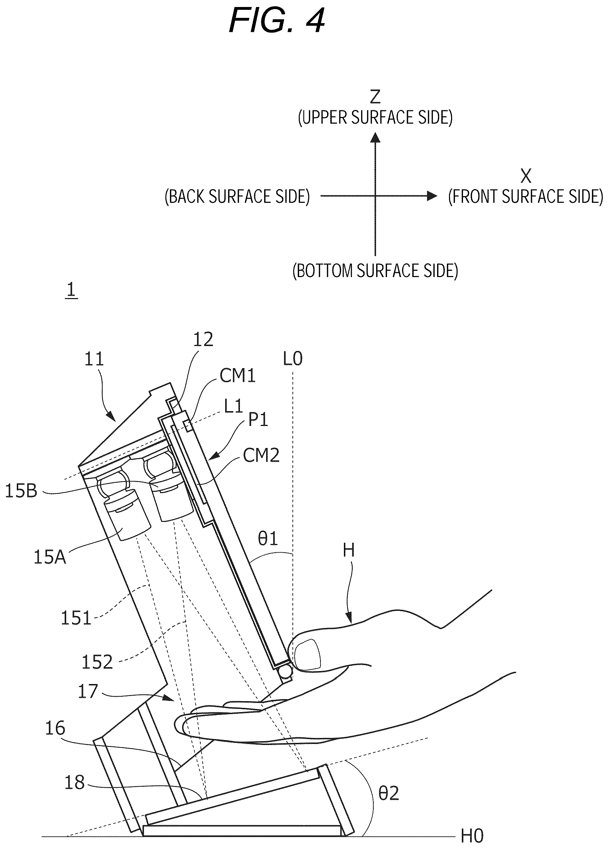

Next, the internal structure of the housing 11 will be described with reference to FIG. 4. FIG. 4 is a left side perspective view of the biometric authentication device 1. The biometric authentication device 1 illustrated in FIG. 4 is a perspective view of the inside of the housing 11 of the biometric authentication device 1 viewed from an arrow V2 illustrated in FIG. 1.

The housing 11 includes two lightings 15A and 15B in a depth direction of the housing 11 (a direction from the front surface side to the back surface side). The number of lightings may be at least one.

Each of the lightings 15A and 15B illuminates the fingertip of the user inserted into the imaging region 17A of the housing internal space 17. Accordingly, the second camera CM2 of the terminal device P1 can acquire the finger image more suitable for the biometric authentication.

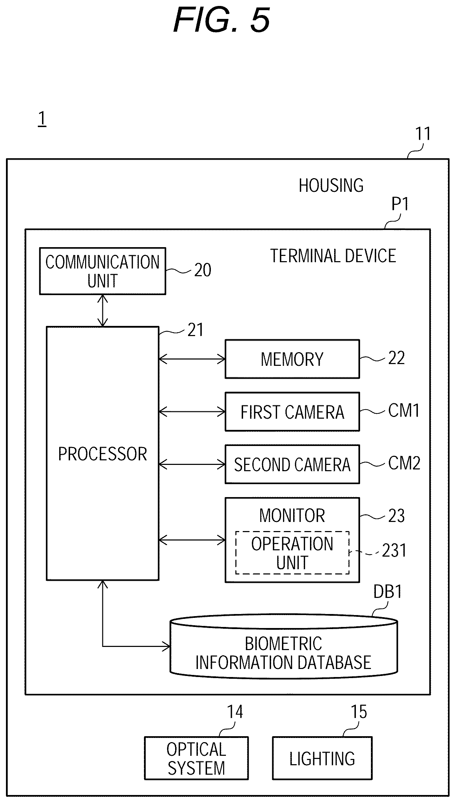

Next, an internal structure example of the terminal device P1 will be described with reference to FIG. 5. FIG. 5 is a block diagram illustrating an internal configuration example of the biometric authentication device 1 according to Embodiment 1.

The internal structure of the biometric authentication device 1 illustrated in FIG. 5 is an example and is not limited thereto. For example, a biometric information database DB1 may be realized by an external storage medium connected to the terminal device P1 so as to be capable of data communication. Further, for example, the monitor 23 may be realized by an external monitor connected to the terminal device P1 so as to be capable of data communication.

The terminal device P1 includes a communication unit 20, a processor 21, a memory 22, the first camera CM1 (an example of a first imaging unit), the second camera CM2 (an example of a second imaging unit), the monitor 23, and the biometric information database DB1. The communication unit 20 is not essential and may be omitted.

The communication unit 20 may be connected to another terminal device installed at the same place or another place sharing information on users such as the same store or an affiliated store so as to be able to perform wireless communication or wired communication. The wireless communication here is, for example, short-range wireless communication such as Bluetooth (registered trademark) or NFC (registered trademark), or communication via a wireless local area network (LAN) such as Wi-Fi (registered trademark).

The processor 21 is implemented by, for example, a central processing unit (hereinafter referred to as "CPU"), a graphics processing unit (hereinafter referred to as "GPU"), or a field programmable gate array (hereinafter referred to as "FPGA"), and performs various types of processing and control in cooperation with the memory 22. Specifically, the processor 21 refers to a program and data stored in the memory 22 and performs the program to implement various functions such as capturing a finger image and a face image and acquiring biometric information (a feature related to a fingerprint, a feature related to a face, and the like).

When acquisition of biometric information or start of biometric authentication is requested by a user operation, the processor 21 as an example of an acquisition unit generates and outputs a control instruction for causing the first camera CM1 and the second camera CM2 to start capturing images of a body part. The processor 21 acquires the captured image output from each of the first camera CM1 and the second camera CM2.

The processor 21 detects and cuts out a region in which the face or a part of the face (for example, eyes, a nose, a mouth, or the like) of the user is shown from the face image output from the first camera CM1, in which the image of the face of the user is captured, and generates the face image. When the biometric information used for biometric authentication is an iris or the like, the processor 21 may detect and cut out, from the captured image, a region in which a body part (for example, an eye) corresponding to the biometric information is shown.

The processor 21 calculates an evaluation value indicating whether a face (for example, eyes, a nose, a mouth, or the like) shown in the generated face image is in focus, that is, whether the face image is an image suitable for face authentication. The processor 21 determines whether the calculated evaluation value is equal to or greater than a first threshold value, and when it is determined that the evaluation value is equal to or greater than the first threshold value, performs face registration or face authentication based on the face image or a feature (hereinafter, referred to as a "face feature") indicating the personality of the user extracted from the face image.

When the processor 21 proceeds to face authentication processing, the processor 21 collates the extracted face feature with each of face features of a plurality of users registered in advance in the biometric information database DB1 to perform the face authentication.

The processor 21 detects a fingertip region including a first joint of at least one fingertip from a captured image obtained by capturing an image of the entire hand or at least one finger of the user output from the second camera CM2. The processor 21 cuts out the detected fingertip region to generate a finger image. When the biometric information used for the biometric authentication is a vein, a palm print, or the like, the processor 21 may detect and cut out, from the captured image, a region in which a body part (for example, a predetermined joint of a finger, a palm, or the like) corresponding to the biometric information is shown.

The processor 21 calculates the evaluation value indicating whether the fingerprint (that is, the body part) shown in the generated finger image is in focus, that is, whether the finger image is an image suitable for fingerprint authentication. The processor 21 determines whether the calculated evaluation value is equal to or greater than a second threshold value, and when it is determined that the evaluation value is equal to or greater than the second threshold value, the processor 21 performs the fingerprint registration or the fingerprint authentication based on the finger image or a feature (hereinafter, referred to as a "fingerprint feature") indicating the individuality of the user extracted from the finger image.

When the processor 21 proceeds to fingerprint authentication processing, the processor 21 collates the extracted fingerprint feature with each of the fingerprint features of the plurality of users registered in advance in the biometric information database DB1 to perform the fingerprint authentication. The processor 21 may collate only the fingerprint feature of the same finger as the finger (for example, a middle finger, an index finger, or the like) corresponding to the extracted fingerprint feature among the fingerprint features of the plurality of users registered in the biometric information database DB1.

The processor 21 generates the notification screen (not illustrated) notifying the user of a registration result or an authentication result based on the registration result of each of the face registration and the fingerprint registration or the authentication result of each of the face authentication and the fingerprint authentication, and outputs and displays the notification screen on the monitor 23. The notification of the registration result or the authentication result may be performed by voice.

The memory 22 includes, for example, a random access memory (hereinafter, referred to as a "RAM") as a work memory used when each processing of the processor 21 is performed, and a read only memory (hereinafter, referred to as a "ROM") storing a program and data defining each operation of the processor 21. The RAM temporarily stores data or information generated or acquired by the processor 21. The program that defines the operation of the processor 21 is written into the ROM. The memory 22 stores the first threshold value related to the evaluation value of the face image, the second threshold value related to the evaluation value of the finger image, and the like.

Each of the first camera CM1 and the second camera CM2 includes at least a lens (not illustrated) and an image sensor (not illustrated). The image sensor is, for example, a solid-state imaging element such as a charged-coupled device (hereinafter referred to as "CCD") or a complementary metal oxide semiconductor (hereinafter referred to as "CMOS"), and converts an optical image formed on an imaging surface into an electric signal. Each of the first camera CM1 and the second camera CM2 starts imaging processing based on a control instruction to start imaging input by the processor 21. Each of the first camera CM1 and the second camera CM2 outputs the captured image to the processor 21.

The monitor 23 is implemented by using, for example, a liquid crystal display (LCD) or an organic electroluminescence (EL). The monitor 23 outputs and displays captured images captured by the first camera CM1 and the second camera CM2, various notification screens generated by the processor 21, or the like.

An operation unit 231 is realized as a touch panel of the monitor 23 described above, can receive a user operation such as an operation of starting an application or an operation of inputting information regarding a user, or an operation by a person such as an employee of a store, and outputs a result of the input operation to the processor 21. The operation unit 231 may include a microphone (not illustrated) and receive a voice input operation based on a voice of a user, an employee of a store, or the like.

The biometric information database DB1 is a so-called storage, and is implemented by a storage medium such as a flash memory, a hard disk drive (HDD), or a solid state drive (SSD). The biometric information database DB1 stores (registers) and manages biometric information such as the face image, the finger image, the face feature, or the fingerprint feature, and information on a user for each user. The biometric information database DB1 may be implemented by an external storage or the like that is externally connected to at least one terminal device P1 and is separately configured.

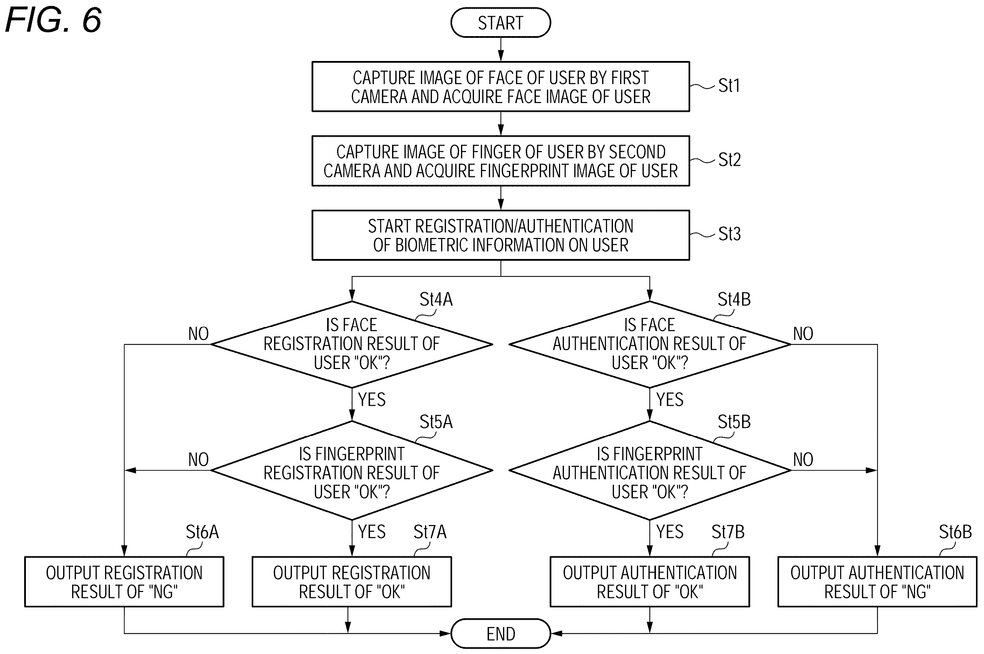

Next, an operation procedure of the terminal device P1 will be described with reference to FIG. 6. FIG. 6 is a sequence diagram illustrating an operational procedure example of the terminal device P1 according to Embodiment 1.

The terminal device P1 receives an operation of designating whether to register or authenticate the biometric information on the user based on the user operation or an employee operation of a facility, a store, or the like where biometric authentication device 1 is installed, and an input operation of information (for example, a name, an age, a gender, an address, a telephone number, a user ID, or the like) on the user, and starts acquisition of the biometric information by each of first camera CM1 and second camera CM2.

The terminal device P1 captures an image of the face of the user by the first camera CM1. The terminal device P1 detects a region in which at least a part of the face of the user is shown from the captured image of the user, and generates the face image obtained by cutting out the region.

Based on the generated face image, the terminal device P1 calculates the evaluation value that is an index indicating whether the face of the user shown in the face image is in focus (that is, whether the face image is a face image from which biometric information suitable for biometric authentication can be acquired). The terminal device P1 determines whether the face image is a registerable finger image based on whether the calculated evaluation value is equal to or greater than the first threshold value indicating that the fingerprint of the user shown in the finger image is in focus.

When it is determined that the calculated evaluation value is equal to or greater than the first threshold value, the terminal device P1 acquires the face image of the user used for registration or authentication of the biometric information (St1). When it is determined that the calculated evaluation value is not equal to or greater than the first threshold value, the terminal device P1 performs re-imaging of the face of the user by the first camera CM1.

The terminal device P1 captures an image of the finger of the user by the second camera CM2. The terminal device P1 detects a region in which at least one fingertip of the user and a part including the first joint of the fingertip are shown from the captured image of the user, and generates the finger image obtained by cutting out the region.

Based on the generated finger image, the terminal device P1 calculates the evaluation value that is an index indicating whether the fingerprint of the user shown in the finger image is in focus (that is, whether the finger image is a finger image from which biometric information suitable for biometric authentication can be acquired). The terminal device P1 determines whether the finger image is a registerable finger image based on whether the calculated evaluation value is equal to or greater than the second threshold value indicating that the fingerprint of the user shown in the finger image is in focus.

When it is determined that the calculated evaluation value is equal to or greater than the second threshold value, the terminal device P1 acquires the finger image of the user used for registration or authentication of the biometric information (St2). When it is determined that the calculated evaluation value is not equal to or greater than the second threshold value, the terminal device P1 performs re-imaging of the finger of the user by the first camera CM1.

The terminal device P1 starts the registration of the biometric information on the user or the performing of the biometric authentication based on each of the plurality of different pieces of acquired biometric information (here, the face image or the finger image) of the user (St3).

### <Registration Processing of Biometric Information>

When registration processing of the biometric information is performed, the terminal device P1 determines whether the user corresponding to the face image is a user who has been stored (registered) in the biometric information database DB1 based on the information on the user input by the user or the employee, and determines whether the face image of the user has been stored (registered) in the biometric information database DB1 (that is, whether the face image is registerable) (St4A).

When it is determined in the processing of step St4A that the user corresponding to the face image is not a user who has been stored (registered) in the biometric information database DB1 and the face image of the user has been not stored (registered) in the biometric information database DB1 (St4A, YES), the terminal device P1 stores (registers) the information on the user and the face image in the biometric information database DB1 in association with each other. The terminal device P1 may extract the face feature indicating the individuality of the user from the face image and store (register) the extracted face feature and the information on the user in the biometric information database DB1 in association with each other.

On the other hand, when it is determined in the processing of step St4A that the user corresponding to the face image is a user who has been stored (registered) in the biometric information database DB1 and the face image of the user has been stored (registered) in the biometric information database DB1 (St4A, NO), the terminal device P1 generates a notification screen (not illustrated) notifying that the registration result of the biometric information on the user is "NG" and outputs the notification screen to the monitor 23 (St6A).

The terminal device P1 determines whether the finger image of the same finger as the finger (for example, the middle finger, the ring finger, or the like) corresponding to the finger image has been stored (registered) in the biometric information database DB1 (that is, whether the finger image is registerable) based on the information on the user input by the user or the employee (St5A).

When it is determined in the processing of step St5A that the finger image of the finger corresponding to the finger image has not been stored (registered) in the biometric information database DB1 (St5A, YES), the terminal device P1 further associates the finger image with the information on the user and stores (registers) the finger image in the biometric information database DB1. The terminal device P1 may extract the fingerprint feature indicating the individuality of the finger of the user from the finger image and store (register) the extracted fingerprint feature in the biometric information database DB1 in association with the information on the user.

The terminal device P1 generates a notification screen (not illustrated) notifying that the registration result of the biometric information on the user is "OK", and outputs the notification screen to the monitor 23 (St7A).

On the other hand, when it is determined in the processing of step St5A that the finger image of the finger corresponding to the finger image has been stored (registered) in the biometric information database DB1 (St5A, NO), the terminal device P1 generates a notification screen (not illustrated) notifying that the registration result of the biometric information on the user is "NG", and outputs the notification screen to the monitor 23 (St6A).

### <Biometric Authentication Processing of Biometric Information>

When biometric authentication processing of the biometric information is performed, the terminal device P1 extracts the face feature of the user from the acquired face image, and collates the extracted face feature with the face feature of each of the plurality of users stored (registered) in the biometric information database DB1 (St4B).

When it is determined in the processing of step St4B that there is a face feature that is the same as or similar to the extracted face feature (St4B, YES), the terminal device P1 determines that a face authentication result of the user is "OK".

On the other hand, when it is determined in the processing of step St4B that there is no face feature that is the same as or similar to the extracted face feature (St4B, NO), the terminal device P1 determines that the face authentication result of the user is "NG", generates a notification screen (not illustrated) notifying that the face authentication result of the user is "NG", and outputs the notification screen to the monitor 23 (St6B). When it is determined that the calculated evaluation value of the face image is not equal to or greater than the first threshold value, the terminal device P1 may determine that the face authentication result of the user is "NG", generate a notification screen (not illustrated) notifying that the face authentication result of the user is "NG", and output the notification screen to the monitor 23.

The terminal device P1 extracts the fingerprint feature of the user from the acquired finger image, and collates the extracted fingerprint feature with the fingerprint feature of each of the plurality of users stored (registered) in the biometric information database DB1 (St5B). The terminal device P1 may perform processing of narrowing down the fingerprint feature to be collated based on finger information corresponding to the finger image. As a result, the terminal device P1 can reduce a processing load required for collation processing of the fingerprint feature and shorten processing time.

In the processing of step St5B, when it is determined that there is a fingerprint feature that is the same as or similar to the extracted fingerprint feature (St5B, YES), it is determined that a fingerprint authentication result of the user is "OK", and the terminal device P1 may generate a notification screen (not illustrated) notifying that the user authentication is "OK" and output the notification screen to the monitor 23 (St18B). When it is determined that both the face authentication result of the face authentication processing and the fingerprint authentication result of the fingerprint authentication processing are "OK" and the authenticated user is the same person, the terminal device P1 may generate a notification screen (not illustrated) notifying that the user authentication is "OK" and output the notification screen to the monitor 23 (St18B).

The terminal device P1 generates a notification screen (not illustrated) notifying that the biometric authentication result of the biometric information on the user is "OK", and outputs the notification screen to the monitor 23 (St7B).

On the other hand, in the processing of step St5B, when it is determined that there is no fingerprint feature that is the same as or similar to the extracted fingerprint feature (St5B, NO), the terminal device P1 determines that the fingerprint authentication result of the user is "NG", generates a notification screen (not illustrated) notifying that the biometric authentication result of the user is "NG", and outputs the notification screen to the monitor 23 (St6B).

As described above, the terminal device P1 according to Embodiment 1 can acquire a plurality of different pieces of biometric information by being placed on the housing 11. In addition, the terminal device P1 can perform a plurality of acquired different biometric authentication.

### (Embodiment 2)

In the biometric authentication device 1 according to Embodiment 1, an example has been described in which the terminal device P1 acquires a plurality of different pieces of biometric information and performs a plurality of different biometric authentication. An example will be described in which the biometric authentication system 100 according to Embodiment 2 acquires a plurality of different pieces of biometric information and performs a plurality of different biometric authentication by different devices.

An internal configuration of the biometric information acquisition device 1A according to Embodiment 2 is similar to an internal configuration of the biometric authentication device 1 according to Embodiment 1. Therefore, in the description of the biometric authentication system 100 according to Embodiment 2, the same reference numerals are given to the same configurations as those of the biometric authentication device 1 (each of the housing 11 and the terminal device P1) described in Embodiment 1, the description thereof will be omitted, and different functions realized by each configuration will be described.

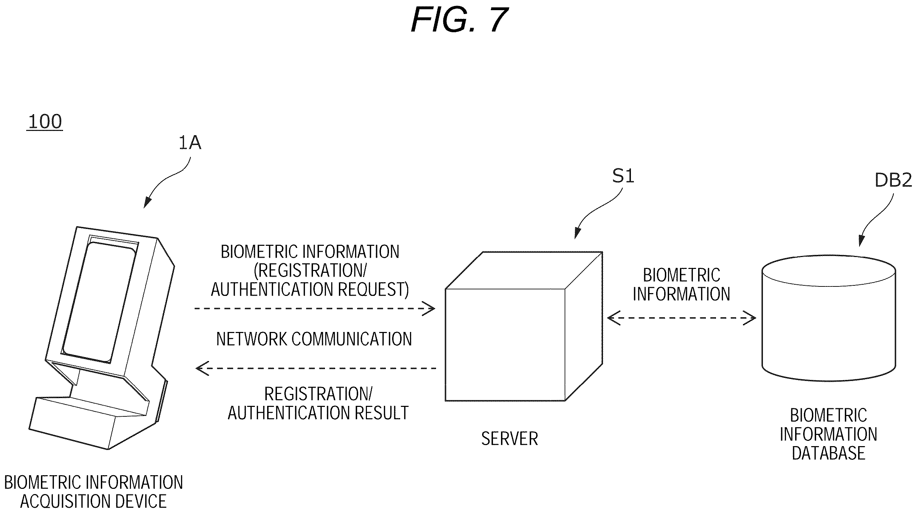

An overall configuration example of the biometric authentication system 100 according to Embodiment 2 will be described with reference to FIG. 7. FIG. 7 is a diagram illustrating the overall configuration example of the biometric authentication system 100 according to Embodiment 2.

The biometric authentication system 100 according to Embodiment 2 includes a biometric information acquisition device 1A including the housing 11 and a terminal device P1A, a server S1, and a biometric information database DB2. The biometric authentication system 100 according to Embodiment 2 acquires a plurality of different pieces of biometric information by a biometric information acquisition device 1A installed in a place where biometric authentication is used, such as a store, and performs a plurality of different biometric authentication by the server S1 connected to the biometric information acquisition device 1A via a network so as to be capable of data communication. The biometric information database DB2 may be configured integrally with the server S1.

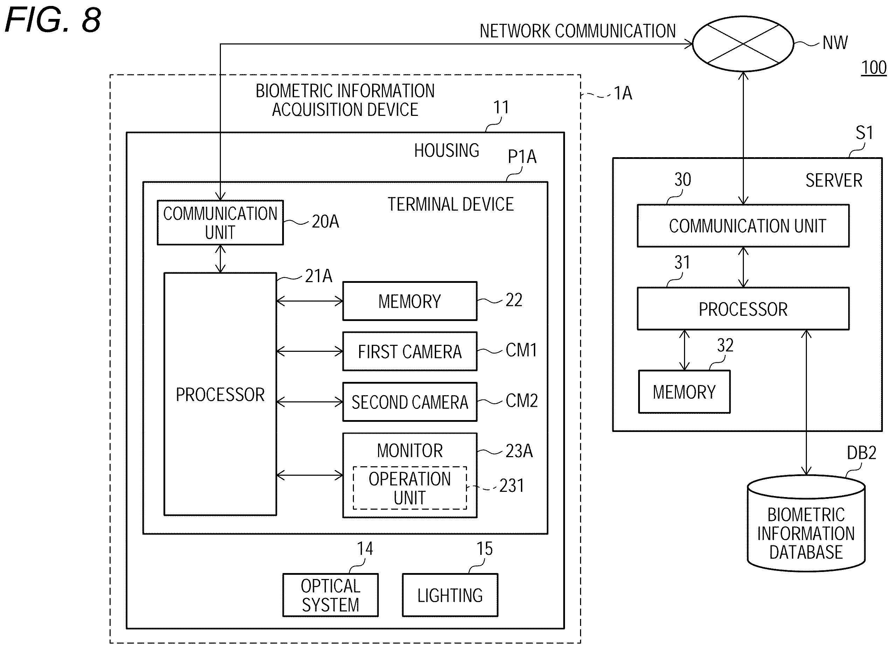

An internal configuration example of a biometric authentication system 100 according to Embodiment 2 will be described with reference to FIG. 8. FIG. 8 is a block diagram illustrating the internal configuration example of the biometric authentication system 100 according to Embodiment 2.

The biometric information acquisition device 1A includes the housing 11 and a terminal device P1A placed on the housing 11. The terminal device P1A includes a communication unit 20A, a processor 21A, the memory 22, the first camera CM1, the second camera CM2, and a monitor 23A.

The communication unit 20A is connected to the server S1 via a network NW so as to be capable of wired communication or wireless communication. The communication unit 20A transmits the biometric information, the control command (the electric signal), and the like output from the processor 21A to the server S1. The communication unit 20A outputs registration result information of the biometric information, the biometric authentication result information, and the like transmitted from the server S1 to the processor 21A.

The processor 21A as an example of an acquisition unit is configured using, for example, CPU, GPU, or FPGA, and performs various processing and control in cooperation with the memory 22. Specifically, the processor 21A refers to the programs and data stored in the memory 22 and performs the programs to realize a function of acquiring biometric information.

The processor 21A cuts out a region in which at least a part of the face of the user is shown from the captured image output from the first camera CM1 to generate the face image. The processor 21A cuts out a region of at least one fingertip in which the first joint of the fingertip is shown from the captured image output from the second camera CM2 to generate the finger image. The processor 21A outputs each of the generated face image and finger image, the information on the user, and a control command for requesting registration of biometric information on the user or the biometric authentication to the communication unit 20A in association with each other, and causes the communication unit 20A to transmit them to the server S1.

The processor 21A may extract the face feature from the generated face image and the fingerprint feature from the finger image, output the extracted face feature and fingerprint feature, the information on the user, and the control command for requesting registration of the biometric information on the user or biometric authentication to the communication unit 20A in association with each other, and transmit them to the server S1.

The processor 21A may generate the finger information corresponding to the finger image or the fingerprint feature and transmit the finger information to the server S1 in association with the finger image or the fingerprint feature. As a result, the biometric authentication system 100 can manage the biometric information for each finger and perform the biometric authentication by identifying the finger.

The monitor 23A is implemented using, for example, an LCD or an organic EL. The monitor 23A outputs and displays the captured images captured by the first camera CM1 and the second camera CM2, various notification screens transmitted from the server S1, or the like.

The server S1 performs the registration processing of the biometric information on the user or the biometric authentication processing of the user based on the biometric information on the user transmitted from the biometric information acquisition device 1A. The server S1 includes a communication unit 30, a processor 31, and a memory 32.

The communication unit 30 is connected to the biometric information acquisition device 1A via the network NW so as to be capable of wired communication or wireless communication. The communication unit 30 is connected to the biometric information database DB2 so as to be capable of wired communication or wireless communication.

The communication unit 30 outputs the biometric information (for example, the face image and the finger image, the face feature, and the fingerprint feature) on the user transmitted from the biometric information acquisition device 1A to the processor 31. The communication unit 30 transmits the information on the biometric information registration result or the biometric authentication result output from the processor 31 to the biometric information acquisition device 1A.

The processor 31 is implemented by, for example, a CPU, a GPU, or a FPGA, and performs various types of processing and control in cooperation with the memory 32. Specifically, the processor 31 refers to the programs and data stored in the memory 22 and performs the programs to realize a function of acquiring the biometric information.

The memory 32 includes, for example, a RAM as a work memory used when each processing of the processor 31 is performed, and a ROM storing a program and data defining an operation of the processor 31. The RAM temporarily stores data or information generated or acquired by the processor 31. The program that defines the operation of the processor 31 is written into the ROM. The memory 32 stores the first threshold value related to the evaluation value of the face image, the second threshold value related to the evaluation value of the finger image, and the like.

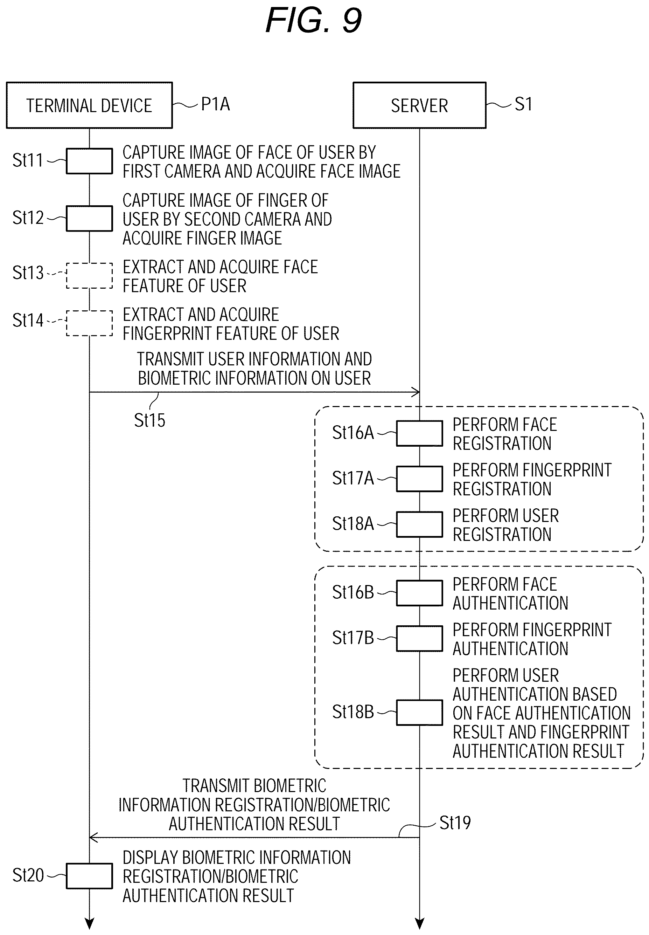

Next, an operation procedure of the biometric authentication system 100 will be described with reference to FIG. 9. FIG. 9 is a sequence diagram illustrating an operational procedure example of the biometric authentication system 100 according to Embodiment 2.

The terminal device P1A of the biometric information acquisition device 1A receives an operation of designating which of the registration of the biometric information on the user and the biometric authentication is to be executed and an input operation of information (for example, a name, an age, a gender, an address, a telephone number, a user ID, or the like) on the user based on the user operation or an employee operation of a facility, a store, or the like in which the biometric authentication device 1 is installed, and starts acquisition of the biometric information by each of the first camera CM1 and the second camera CM2.

The terminal device P1A captures the image of the face of the user by the first camera CM1. The terminal device P1A detects the region in which at least a part of the face of the user is shown from the captured image of the user, and generates the face image obtained by cutting out the region (St11).

Based on the generated face image, the terminal device P1A calculates an evaluation value that is an index indicating whether the face of the user illustrated in the face image is in focus (that is, whether the face image is a face image from which biometric information suitable for biometric authentication can be acquired) (St11). The terminal device P1A determines whether the face image is a registrable face image based on whether the calculated evaluation value is equal to or greater than the first threshold value indicating that the face of the user shown in the face image is in focus (St11).

When it is determined that the calculated evaluation value is equal to or greater than the first threshold value, the terminal device P1A acquires the face image of the user used for registration or authentication of the biometric information (St11). When it is determined that the calculated evaluation value is not equal to or greater than the first threshold value, the terminal device P1A performs re-imaging of the face of the user by the first camera CM1.

The terminal device P1A captures the image of the finger of the user by the second camera CM2. The terminal device P1A detects a region in which at least one finger of the user and a part including the first joint of the finger are shown from the captured image of the user, and generates the finger image obtained by cutting out the region (St12).

Based on the generated finger image, the terminal device P1A calculates the evaluation value that is an index indicating whether the fingerprint of the user shown in the finger image is in focus (that is, whether the finger image is a finger image from which biometric information suitable for biometric authentication can be acquired) (St12). The terminal device P1A determines whether the finger image is a registerable finger image based on whether the calculated evaluation value is equal to or greater than the second threshold value indicating that the fingerprint of the user shown in the finger image is in focus (St12).

When it is determined that the calculated evaluation value is equal to or greater than the second threshold value, the terminal device P1A acquires the finger image of the user used for the registration of the biometric information or the biometric authentication (St12). When it is determined that the calculated evaluation value is not equal to or greater than the second threshold value, the terminal device P1A executes re-imaging of the finger of the user by the first camera CM1.

The terminal device P1A extracts and acquires the face feature from the acquired face image (St13). The terminal device P1 extracts and acquires the fingerprint feature from the acquired finger image (St14). Note that each of the processing of step St13 and step St14 may be omitted when the terminal device P1A acquires each of the face image and the finger image as the biometric information and transmits it to the server S1.

The terminal device P1A generates a control command requesting either the registration of the biometric information or the biometric authentication based on an operation of designating which of the registration of the biometric information on the user and the biometric authentication is to be executed. The terminal device P1A transmits the generated control command, the information on the user, and the biometric information on the user to the server S1 in association with each other (St15).

The server S1 starts the registration of the biometric information on the user or the execution of the biometric authentication based on the control command transmitted from the terminal device P1A. In the following description, an example will be described in which the terminal device P1A transmits each of the face image and the finger image.

### <Registration Processing of Biometric Information>

The server S1 determines whether the user corresponding to the face image is a user who has been stored (registered) in the biometric information database DB2 based on the information on the user, and determines whether the face image of the user has been stored (registered) in the biometric information database DB2 (that is, whether the face image is registerable) (St16A).

When it is determined that the user corresponding to the face image is not a user who has been stored (registered) in the biometric information database DB2 and the face image of the user has been not stored (registered) in the biometric information database DB2, the server S1 stores (registers) the information on the user and the face image in the biometric information database DB2 in association with each other (St16A).

The server S1 determines whether the finger image of the same finger as the finger (for example, the middle finger, the ring finger, or the like) corresponding to the acquired finger image has been stored (registered) in the biometric information database DB2 (that is, whether the finger image is registerable) based on the information on the user input by the user or the employee (St17A).

When it is determined that the finger image of the finger corresponding to the acquired finger image has not been stored (registered) in the biometric information database DB2, the server S1 further associates the finger image with the face image and the information on the user and stores (registers) the finger image in the biometric information database DB2 (St18A).

When the registration of each of the face image and the finger image is completed, the server S1 generates information on a biometric information registration result notifying that the registration result of the biometric information on the user is "OK" (St18A). When only one of the face image and the finger image is registered, the server S1 may generate information on the biometric information registration result notifying that only one of the face image and the finger image is registered as the registration result of the biometric information on the user. Thus, the server S1 can accept additional registration and update registration of the face image and the finger image.

When it is determined that both the face image and the finger image have been stored (registered) in the biometric information database DB2, the server S1 generates information on the biometric information registration result notifying that the registration result of the biometric information on the user is "NG" (St18A).

### <Biometric Authentication Processing of Biometric Information>

The server S1 extracts the face feature from the acquired face image based on the control command transmitted from the terminal device P1A. The server S1 refers to the biometric information database DB2 and collates the extracted face feature with the face feature of each of the plurality of users who have been stored (registered) in the biometric information database DB2 (St16B).

When it is determined that there is a face feature that is the same as or similar to the extracted face feature, the server S1 determines that the face authentication result of the user is "OK" (St16B).

On the other hand, when it is determined that there is no face feature that is the same as or similar to the acquired face feature, the server S1 determines that the face authentication result of the user is "NG" (St16B). In such a case, the server S1 may omit the processing of step St17A.

The server S1 extracts the fingerprint feature from the acquired finger image based on the control command transmitted from the terminal device P1A. The server S1 refers to the biometric information database DB2 and collates the acquired fingerprint feature with the fingerprint feature of each of the plurality of users who have been stored (registered) in the biometric information database DB2 (St17B). Note that the server S1 may perform the processing of narrowing down the fingerprint feature to be matched based on the finger information transmitted from the terminal device P1A or the finger information corresponding to the finger image at the time of fingerprint feature extraction. As a result, the terminal device P1 can reduce a processing load required for collation processing of the fingerprint feature and shorten processing time.

When it is determined that there is a fingerprint feature that is the same as or similar to the extracted fingerprint feature, the server S1 determines that the fingerprint authentication result of the user is "OK" (St17B).

On the other hand, when it is determined that there is no fingerprint feature that is the same as or similar to the extracted fingerprint feature, the server S1 determines that the fingerprint authentication result of the user is "NG" (St17B).

When it is determined that both the face authentication result of the face authentication processing and the fingerprint authentication result of the fingerprint authentication processing are "OK" and the authenticated user is the same person, the server S1 determines that the user authentication is "OK" and generates information on the biometric authentication result notifying that the biometric authentication result is "OK" (St18B).

On the other hand, when it is determined that at least one of the face authentication result of the face authentication processing and the fingerprint authentication result of the fingerprint authentication processing is "NG", the server S1 determines that the user authentication is "NG" and generates information on the biometric authentication result notifying that the biometric authentication result is "NG" (St18B).

The server S1 transmits the information on the registration result of the biometric information on the user or the information on the biometric authentication result to the terminal device P1A (St19).

The terminal device P1A generates a notification screen (not illustrated) notifying the biometric information registration result or the biometric authentication result based on the information on the registration result of the biometric information on the user or the information on the biometric authentication result transmitted from the server S1, and outputs the notification screen to the monitor 23A to display the notification screen thereon (St20).

As described above, the biometric authentication system 100 according to Embodiment 2 can register a plurality of different pieces of biometric information and execute a plurality of different biometric authentication using a respective one of the plurality of different pieces of biometric information.

As described above, the biometric authentication device 1 according to Embodiment 1 and the biometric information acquisition device 1A according to Embodiment 2 are examples of a biometric information acquisition device, and each include the housing 11, the first camera CM1 (an example of a first imaging unit) configured to capture an image of a first body part of a user (an example of a subject) in a contactless state, the second camera CM2 (an example of a second imaging unit) configured to capture an image of a second body part different from the first body part of the subject in the contactless state, and the processor 21 (an example of an acquisition unit) configured to acquire a first captured image captured by the first camera CM1 and a second captured image captured by the second camera CM2. The housing 11 includes the opening portion 16 (an example of a first opening portion) that is formed by cutting out a part of the housing 11 to form the imaging region 17A (an example of an imaging space) in which the image of the second body part is captured, the lightings 15A and 15B that illuminate the imaging region 17A inside the housing 11, and the optical system 14 (an example of an optical path changing unit) that changes an optical path of light reflected by the second body part inside the imaging region 17A and causes the light to be incident on the second camera CM2.

Accordingly, the biometric authentication device 1 according to Embodiment 1 and the biometric information acquisition device 1A according to Embodiment 2 can acquire a plurality of different pieces of biometric information by the terminal devices P1 and P1A that each are placed on the housing 11 and include two cameras (the first camera CM1 and the second camera CM2). The biometric information referred to here may be a captured image or a feature extracted from the captured image.

In the biometric authentication device 1 according to Embodiment 1 and the biometric information acquisition device 1A according to Embodiment 2, the first camera CM1 captures the image of the first body part outside the housing 11. Thus, the biometric authentication device 1 according to Embodiment 1 and the biometric information acquisition device 1A according to Embodiment 2 can acquire a plurality of different pieces of biometric information inside and outside the housing 11, respectively.

In the biometric authentication device 1 according to Embodiment 1 and the biometric information acquisition device 1A according to Embodiment 2, the optical axis L1 of the first camera CM1 has an angle of 15° to 23° with respect to the installation surface H0 of the housing 11 in a side view of the housing 11 (for example, as viewed from the arrows V1 and V2). Accordingly, the biometric authentication device 1 according to Embodiment 1 and the biometric information acquisition device 1A according to Embodiment 2 can capture an image of at least a part of the face of the user located diagonally above the front of the housing 11 by the first camera CM1 and an image of at least a part of the finger of the user inserted into the housing 11 by the second camera CM2.

In the biometric authentication device 1 according to Embodiment 1 and the biometric information acquisition device 1A according to Embodiment 2, the first camera CM1 captures the image of at least a part of the face of the user, and the second camera CM2 captures the image of at least a part of the finger of the user. Accordingly, the biometric authentication device 1 according to Embodiment 1 and the biometric information acquisition device 1A according to Embodiment 2 can capture an image of at least a part of the face of the user located diagonally above the front of the housing 11 by the first camera CM1 and an image of at least a part of the finger of the user inserted into the housing 11 by the second camera CM2.

In the biometric authentication device 1 according to Embodiment 1 and the biometric information acquisition device 1A according to Embodiment 2, the housing 11 further includes the recess 12 (an example of the attachment and detachment portion) to which the terminal devices P1 and P1A (an example of an imaging device) is attachable and detachable on the front surface side of the housing 11. The terminal devices P1 and P1A each include the first camera CM1, the second camera CM2, and the processor 21. Accordingly, the biometric authentication device 1 according to Embodiment 1 and the biometric information acquisition device 1A according to Embodiment 2 can acquire a plurality of different pieces of biometric information by placing the terminal devices P1 and P1A on the housing 11.

In the biometric authentication device 1 according to Embodiment 1 and the biometric information acquisition device 1A according to Embodiment 2, the recess 12 has the opening 13 (an example of the second opening portion). The optical system 14 causes the light whose optical path has been changed to be incident on the second camera CM2 through the opening 13. Accordingly, the biometric authentication device 1 according to Embodiment 1 and the biometric information acquisition device 1A according to Embodiment 2 can adjust the optical path length of the light incident on the second camera CM2 (a distance between the finger of the user and the image sensor of the second camera CM2) to be within the depth of field of the second camera CM2, and can further reduce a housing height of the housing 11 in the upper-lower direction (Z-axis direction).

In the biometric authentication device 1 according to Embodiment 1 and the biometric information acquisition device 1A according to Embodiment 2, the optical system 14 is a prism. Thus, the biometric authentication device 1 according to Embodiment 1 and the biometric information acquisition device 1A according to Embodiment 2 can adjust the optical path length of the light incident on the second camera CM2 (the distance between the finger of the user and the image sensor of the second camera CM2).

In the biometric authentication device 1 according to Embodiment 1 and the biometric information acquisition device 1A according to Embodiment 2, the optical system 14 is a mirror. Thus, the biometric authentication device 1 according to Embodiment 1 and the biometric information acquisition device 1A according to Embodiment 2 can adjust the optical path length of the light incident on the second camera CM2 (the distance between the finger of the user and the image sensor of the second camera CM2).

In the biometric authentication device 1 according to Embodiment 1 and the biometric information acquisition device 1A according to Embodiment 2, the opening portion 16 is formed such that an opening height decreases from the front surface toward the back surface of the housing 11 in a side view of the housing 11 (for example, as viewed from the arrows V1 and V2). As a result, the biometric authentication device 1 according to Embodiment 1 and the biometric information acquisition device 1A according to Embodiment 2 can facilitate insertion of the user's finger from the front surface side of the housing 11 toward the housing internal space 17 (the imaging region 17A).

In the biometric authentication device 1 according to Embodiment 1 and the biometric information acquisition device 1A according to Embodiment 2, the opening portion 16 is formed such that an angle formed by the installation surface H0 of the housing 11 and the opening lower surface 18 of the opening portion 16 decreases from the front surface toward the back surface of the housing 11 in a side view of the housing 11 (for example, as viewed from the arrows V1 and V2). As a result, the biometric authentication device 1 according to Embodiment 1 and the biometric information acquisition device 1A according to Embodiment 2 can facilitate insertion of the user's finger from the front surface side of the housing 11 toward the housing internal space 17 (the imaging region 17A).

Although various embodiments have been described above with reference to the accompanying drawings, the present disclosure is not limited thereto. It is apparent to those skilled in the art that various modifications, corrections, substitutions, additions, deletions, and equivalents can be conceived within the scope described in the claims, and it is understood that such modifications, corrections, substitutions, additions, deletions, and equivalents also fall within the technical scope of the present disclosure. In addition, components in the various embodiments described above may be combined freely in a range without deviating from the spirit of the invention.

The present application is based on a Japanese Patent Application (Japanese Patent Application No. 2022-210339) filed on December 27, 2022, and the contents thereof are incorporated herein by reference.

### INDUSTRIAL APPLICABILITY

The present disclosure is useful as a presentation of a biometric information acquisition device that assists in acquisition of a plurality of different pieces of biometric information suitable for biometric authentication in a contactless state.

### REFERENCE SIGNS LIST

1 biometric authentication device
1A biometric information acquisition device
11 housing
12 recess
13 opening
14 optical system
15A, 15B lighting
16 opening portion
17 housing internal space
17A imaging region
18 opening lower surface
20, 30 communication unit
21, 31 processor
22, 32 memory
23, 23A monitor
CM1 first camera
CM2 second camera
DB1, DB2 biometric information database
S1 server
P1, P1A terminal device

## Claims

1. A biometric information acquisition device comprising:
a housing;
a first imaging unit that captures an image of a first body part of a subject in a contactless state;
a second imaging unit that captures an image of a second body part different from the first body part of the subject in the contactless state; and
an acquisition unit that acquires a first captured image captured by the first imaging unit and a second captured image captured by the second imaging unit, wherein
the housing includes
a first opening portion that is formed by cutting out a part of the housing to form an imaging space in which the image of the second body part is captured,
a lighting that illuminates the imaging space inside the housing, and
an optical path changing unit that changes an optical path of light reflected by the second body part inside the imaging space and causes the light to be incident on the second imaging unit.

2. The biometric information acquisition device according to claim 1, wherein
the first imaging unit captures the image of the first body part outside the housing.

3. The biometric information acquisition device according to claim 1, wherein
an optical axis of the first imaging unit has an angle of 15° to 23° with respect to an installation surface of the housing in a side view of the housing.

4. The biometric information acquisition device according to claim 1, wherein
the first imaging unit captures an image of at least a part of a face of the subject, and
the second imaging unit captures an image of at least a part of a finger of the subject.

5. The biometric information acquisition device according to claim 1, wherein
the housing further includes an attachment and detachment portion to which an imaging device is attachable and detachable on a front surface side of the housing, and
the imaging device includes the first imaging unit, the second imaging unit, and the acquisition unit.

6. The biometric information acquisition device according to claim 5, wherein
the attachment and detachment portion has a second opening portion, and
the optical path changing unit causes the light whose optical path has been changed to be incident on the second imaging unit through the second opening portion.

7. The biometric information acquisition device according to claim 1, wherein
the optical path changing unit is a prism.

8. The biometric information acquisition device according to claim 1, wherein
the optical path changing unit is a mirror.

9. The biometric information acquisition device according to claim 1, wherein
the first opening portion is formed such that an opening height decreases from a front surface toward a back surface of the housing in a side view of the housing.

10. The biometric information acquisition device according to claim 1, wherein
the first opening portion is formed such that an angle formed by an installation surface of the housing and an opening lower surface of the first opening portion decreases from a front surface toward a back surface of the housing in a side view of the housing.
